# EUROPEAN PATENT APPLICATION

(11) **EP 4 316 383 A1**
(43) Date of publication of application: **07.02.2024**
(21) Application number: 22188662.5
(22) Date of filing: 04.08.2022
(51) Int. Cl.: A61B 8/08, A61N 7/00

(54) **APPARATUS AND METHOD FOR PERFORMING NON-INVASIVE THERMODILUTION**

(71) Applicant: Koninklijke Philips N.V., 5656 AG Eindhoven (NL)
(72) Inventor: VAN LIESHOUT, Ron Martinus Laurentius, Eindhoven (NL); BERA, Deep, Eindhoven (NL); SHULEPOV, Sergei Y., 5656AG Eindhoven (NL)
(74) Representative: Philips Intellectual Property & Standards

(57) **Abstract**

Provided is an apparatus and method for performing non-invasive thermodilution and creating thermodilution curves, using high-intensity focused ultrasound (HIFU) and ultrasonic thermometry. The apparatus of the present disclosure includes an ultrasonic transducer system comprising one or more ultrasonic transducers or transducer arrays and a processor communicatively coupled to the ultrasonic transducer system. The ultrasonic transducer system is configured to locally increase the temperature of blood of a subject using HIFU and measure a corresponding change in temperature of the blood downstream using ultrasonic thermometry. The processor is configured to create a thermodilution curve based on receiving signals pertaining to the measure of the change in temperature from the ultrasound transducer system.

## Description

### FIELD OF THE INVENTION

The present disclosure generally relates to thermodilution apparatuses and techniques. More particularly, embodiments herein relate to an apparatus and method for performing non-invasive thermodilution and creating thermodilution curves, using high-intensity focused ultrasound (HIFU) and ultrasonic thermometry.

### BACKGROUND OF THE INVENTION

Blood flow measurement is both physiologically and clinically important. Peripheral circulation often decreases under pathological conditions. Blood flow in humans is largely distributed from large vessels (arteries) to peripheral areas and back to large vessels (veins). Both the velocity of flow and vessel dimensions change by a large factor from peripheral to large vessels. Blood flow and cardiac output are important parameters to measure when patients are hemodynamically unstable.

In order to measure blood flow and cardiac output, a thermodilution method is employed that involves injection of a definite amount of fluid at a certain temperature into the bloodstream, and the corresponding downstream temperature change is recorded. A cold fluid is often used as an indicator in the thermodilution method because cold fluid is less harmful to the blood and tissue when compared to a hot fluid. For example, a volume of ∼10 ml of cold saline or isotonic dextrose solution near 0°C is commonly used for cardiac output measurement in a human adult. The injected cold indicator is mixed and diluted in the warm bloodstream and causes a slight temperature to decrease in the blood downstream.

The thermodilution method has several advantages; that is, the indicator has no toxicity, and so measurements can be performed repeatedly, the dilution curve can be easily recorded by a thermistor placed in the vessel, and the recirculation component is sufficiently small that integration of the dilution curve can be performed accurately. The fundamental assumption of the indicator dilution method that the indicator should not leak out from the vascular system between the injection and detection sites is not perfectly valid because heat can dissipate across the vessel wall. This effect is insignificant in large vessels due to the small ratio of surface area to volume per unit length. Thus, the thermodilution method is more appropriate for flow measurements in large than in smaller vessels.

Hitherto known thermodilution techniques employ a specially designed catheter, called the Swan-Ganz thermodilution catheter, which is widely used for cardiac output measurements. Conventionally, the catheter is introduced from a peripheral vein into the pulmonary artery through the right ventricle. A bolus of cold saline or dextrose solution is injected into the right atrium, mixing occurs in the right atrium and the right ventricle, and the resultant temperature decrease is detected by the thermistor placed in the pulmonary artery.

A different approach to the thermodilution method using intravascular heating was also attempted. An electric heater can be used to transfer heat into the bloodstream. In this study, heating wire was wound around a standard thermodilution catheter. When a sinusoidal thermal signal of 0.02 Hz with an average power of 4 W was applied to the right ventricle of sheep, and blood temperature change in the pulmonary artery was simultaneously detected, cardiac output could be measured over the range of 1.8-9.5 1/min. A correlation coefficient of 0.977 was obtained between the heating method and the standard rapid injection thermodilution measurements.

The above-described techniques, however, are invasive and may be associated with the onset of complications.

Pulse Indicator Continuous Cardiac Output (PiCCO) is also a thermodilution measurement which requires a complex and invasive setup.

Although thermodilution provides important clinical information, it is an invasive method associated with numerous complications. Thermodilution can also induce blocks in the right bundle branch and even in the complete heart. Moreover, the use of a fluid bolus has an impact on the fluid management of the patient. Fluid overload of a patient needs to be prevented and this has an impact on the amount of fluid boluses that may be administered to a patient. Therefore, the current thermodilution methods cannot be used in a high measurement frequency setting. The current gold standard requires a fluid bolus that creates fluid overload in patients.

By using non-invasive methods to measure the thermodilution curve (which is the gold standard) the acceptability of the technology may be improved. However, the ultrasound technologies currently available to measure cardiac output, still require skilled operators and are not continuous. In addition, these thermodilution methods are still seen as the gold standard and clinicians are used to interpret and process the information obtained from these methods.

Therefore, there exists a need for a system and method that is aimed at making the thermodilution methods non-invasive without the need for a skilled operator, at the same time addressing the drawbacks of existing thermodilution techniques.

Limitations and disadvantages of conventional and traditional approaches will become apparent to one of ordinary skill in the art, through comparison of described systems with some aspects of the present disclosure, as set forth in the remainder of the present application and with reference to the drawings.

### SUMMARY OF THE INVENTION

A claimed solution rooted in computer technology overcomes problems specifically arising in the realm of computer technology when performing thermodilution and creating thermodilution curves, to measure blood flow and cardiac output (CO) of subjects.

An appartus and method for performing thermodilution is provided substantially as shown in and/or described in connection with, at least one of the figures, as set forth more completely in the claims.

These and other features and advantages of the present disclosure may be appreciated from a review of the following detailed description of the present disclosure, along with the accompanying figures in which like reference numerals refer to like parts throughout.

In the claimed solution of the present disclosure, the apparatus for performing non-invasive thermodilution includes an ultrasonic transducer system. The ultrasonic transducer system includes one or more ultrasonic transducers or transducer arrays. The ultrasonic transducer system is configured to: locally increase the temperature of blood of a subject using high-intensity focused ultrasound (HIFU) and measure a corresponding change in temperature of the blood downstream using ultrasonic thermometry. A processor communicatively coupled to the ultrasonic transducer system is configured to create a thermodilution curve based on receiving signals pertaining to the measure of the change in temperature from the ultrasound transducer system.

In accordance with an embodiment, the ultrasonic transducer system is configured to administer a bolus into the bloodstream of the subject to locally increase the temperature of the blood using HIFU. The bolus in some embodiments can be, but need not be limited to, a cold saline solution or a dextrose solution. A temperature of the bolus administered is different from a temperature of the bloodstream of the subject.

In some embodiments, the ultrasonic transducer system measures a blood flow rate of the subject. The processor is configured to derive a cardiac output of the subject from the thermodilution curve based on a thermal dissipation of the bolus due to bloodstream in the heart of the subject.

In accordance with an embodiment, the processor is configured to analyze the thermodilution curve using a frequency profile based on signal processing. The techniques used to analyze the thermodilution curve may include, but are not limited to, a Fast Fourier Transform (FFT) signal processing and an impulse response function.

In some embodiments, a thermodilution profile amplitude of the thermodilution curve is increased by placing an HIFU focal point and a thermometry measurement point closer to each other.

In some other embodiments, a frequency of temperature increase and dissipation (also known as 'temperature injection frequency') is chosen such that the temperature of the blood is increased directly after complete thermal dissipation of the bolus, to obtain a lower cardiac output that changes a frequency profile of the thermodilution curve. The processor is configured to detect an increase in a mean signal from the ultrasonic transducer system due to the cardiac output being lower with a same frequency of temperature increase of blood and the thermal dissipation of the bolus being below a baseline. Upon detecting an increase in the mean signal, the processor is configured to shift the frequency of temperature increase and dissipation.

A principal object of the present disclosure is to provide a non-invasive thermodilution method to avoid risks and serious complications when used in a clinical setting. As fluids are not used to create a temperature difference, there is no limit to the number of times the thermodilution technique of the present disclosure is operated. Using the HIFU heating and ultrasound thermometry or thermography in a repeated way, the resulting thermodilution curve reflects a frequency profile, which can be analyzed using techniques such as, but not limited to, FFT signal processing and impulse response methods. Furthermore, by choosing the temperature injection frequency such that the temperature increase is directly after the complete heat dissipation, a lower cardiac output (CO) changes the frequency profile.

Furthermore, by removing the need for the thermistor and replacing it with ultrasound thermography, the system is safer, easier to use, and less costly. The thermodilution profile is also of a lower amplitude compared to the currently known cold saline bolus methods.

It should be appreciated that all combinations of the foregoing concepts and additional concepts discussed in greater detail below (provided such concepts are not mutually inconsistent) are contemplated as being part of the subject matter disclosed herein. In particular, all combinations of claimed subject matter appearing at the end of this disclosure are contemplated as being part of the subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

### BRIEF DESCRIPTION OF THE DRAWINGS

The various advantages of the embodiments will become apparent to one skilled in the art by reading the following specification and appended claims, and by referencing the following drawings, in which:
FIG. 1 is a block diagram illustrating a system implementing an apparatus as described herein for performing non-invasive thermodilution in accordance with an exemplary embodiment of the disclosure.
FIG. 2 is a block diagram illustrating an apparatus for performing non-invasive thermodilution in accordance with an exemplary embodiment of the disclosure.
FIG. 3 and FIG. 4 illustrate thermodilution curves in accordance with various embodiments of the disclosure.
FIG. 5 is a flowchart illustrating a method for performing non-invasive thermodilution in accordance with an exemplary embodiment of the disclosure.

### DETAILED DESCRIPTION OF EMBODIMENTS

As will be described in greater detail below, in some implementations discussed herein, an apparatus and method may advantageously be used to address parts of the challenges in performing non-invasive thermodilution for measuring blood flow and cardiac output (CO) of subjects.

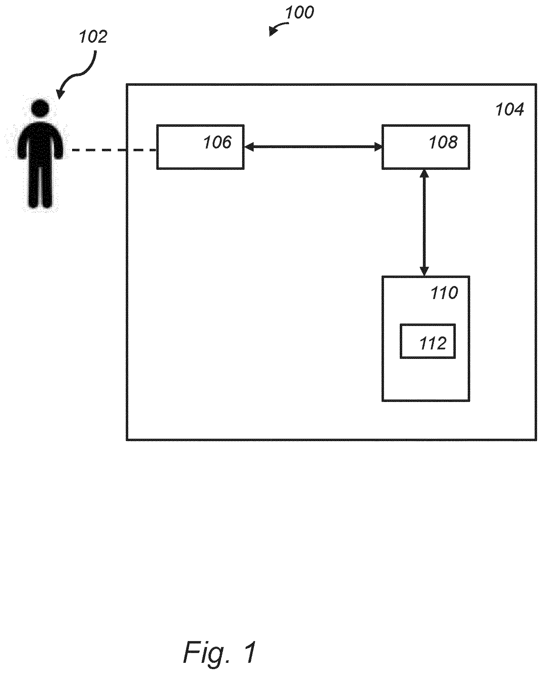

FIG. 1 is a block diagram illustrating a system implementing an apparatus as described herein for performing non-invasive thermodilution in accordance with an exemplary embodiment of the disclosure. Referring to FIG. 1, there is shown a system 100 that includes a subject 102, an apparatus 104 that includes an ultrasonic transducer system 106, a processing system 108, a user interface 110 and a thermodilution curve 112 displayed on the user interface 110.

The ultrasound transducer system 106 includes one or more ultrasonic transducers or transducer arrays that may comprise suitable logic, circuitry, interfaces and/or code that may be operable to transmit ultrasound radiations or vibrations into the subject 102, to locally increase the temperature of blood of the subject 102 using high-intensity focused ultrasound (HIFU). The ultrasound transducer system 106 is further configured to measure a corresponding change in temperature of the blood downstream using ultrasonic thermometry.

The processing system 108 is communicatively coupled to the ultrasonic transducer system 106 and the user interface 110. The user interface 110 may be implemented via one or more form-factor devices that may include, but are not limited to, a smart phone, a tablet, a laptop, a workstation, and/or the like.

The ultrasound processing system 106, the processing system 108 and the user interface 110 may be in communication with one another via communication technologies that may include, but are not limited to, Internet-based communication, cloud-based communication, wired communication, wireless communication, the like, and/or combinations thereof.

The processing system 108 may comprise suitable logic, interfaces, and/or code that may be configured to create a thermodilution curve 112 that is displayed on the user interface 110, based on receiving signals pertaining to the measure of the change in temperature from the ultrasound transducer system 106.

In operation, the ultrasonic transducer system 106 is configured to administer a bolus into the bloodstream of the subject 102 to locally increase the temperature of the blood using HIFU. The bolus may be, but is not limited to, a cold saline solution or a dextrose solution. A temperature of the bolus is different from a temperature of the bloodstream of the subject 102. The ultrasonic transducer system 106 is further configured to measure a corresponding change in temperature of the blood downstream using ultrasonic thermometry.

In some embodiments, the ultrasonic transducer system 106 measures a blood flow rate of the subject 102.

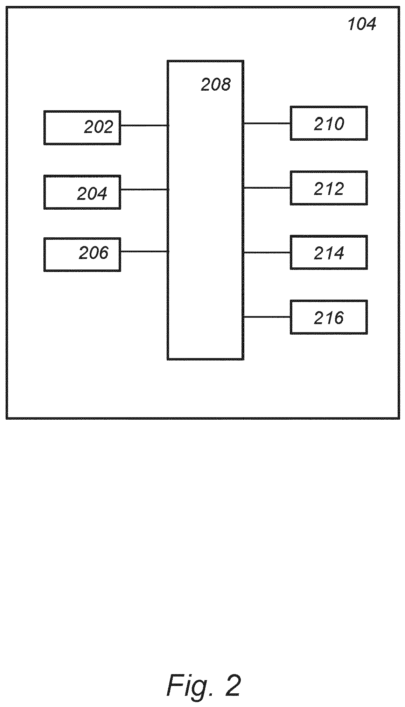

FIG. 2 is a block diagram illustrating an apparatus for performing non-invasive thermodilution in accordance with an exemplary embedment of the disclosure. Referring to FIG. 2, there is shown the apparatus 104 that includes a memory 202, a user interface 204, a processor 206, a communications unit 208, a transducer interface 210, a measurement component 212, a thermodilution curve creation component 214 and an analysis component 216.

The memory 202 may include, but is not limited to, one or more memory devices, persistent storage, computer-readable storage media, random access memory (RAM) and cache memory. In general, the memory 202 may include any suitable volatile or non-volatile computer-readable storage media. The memory 202 may comprise suitable logic, and/or interfaces, that may be configured to store instructions (for example, computer-readable program code) that can implement various aspects of the present disclosure.

The memory 202 is communicatively coupled to the user interface 204 and the processor 206.

The user interface 204 may be implemented via one or more form-factor devices that may include, but are not limited to, a smart phone, a tablet, a laptop, a workstation, and/or the like.

The processor 206 may comprise suitable logic, interfaces, and/or code that may be configured to execute the instructions stored in the memory 202 to implement various functionalities of the apparatus 104 in accordance with various aspects of the present disclosure. The processor 206 may be further configured to communicate with various modules of the apparatus 104 via the communications unit 208.

The communications unit 208 may be configured to transmit data between modules, engines, databases, memories, and other components of the apparatus 104 for use in performing the functions discussed herein. The communications unit 208 may include one or more communication types and utilize various communication methods for communication within the apparatus 104.

The transducer interface 210 couples the ultrasonic transducer system 106 with the apparatus 104. The ultrasound transducer system 106 includes one or more ultrasonic transducers or transducer arrays that may comprise suitable logic, circuitry, interfaces and/or code that may be operable to transmit ultrasound radiations or vibrations into the subject 102, to locally increase the temperature of blood of the subject 102 using high-intensity focused ultrasound (HIFU).

The measurement component 212 may comprise suitable logic, interfaces, and/or code that may be configured to measure a corresponding change in temperature of the blood downstream using ultrasonic thermometry.

The thermodilution curve creation component 214 may comprise suitable logic, interfaces, and/or code that may be configured to create the thermodilution curve 112 that is displayed on the user interface 204, based on receiving signals pertaining to the measure of the change in temperature from the ultrasound transducer system 106.

The analysis component 216 may comprise suitable logic, interfaces, and/or code that may be configured to derive a cardiac output (CO) of the subject 102 from the thermodilution curve 112 based on a thermal dissipation of the bolus due to bloodstream in the heart of the subject 102. The analysis component 216 is configured to analyze the thermodilution curve 112 using a frequency profile based on signal processing, using, for example, a Fast Fourier Transform (FFT) signal processing, an impulse response function and the like.

In an embodiment, a thermodilution profile amplitude of the thermodilution curve 112 is increased by placing an HIFU focal point and a thermometry measurement point closer to each other.

In accordance with various embodiments, a frequency of temperature increase and dissipation (also known as 'temperature injection frequency') is chosen such that the temperature of the blood is increased directly after complete thermal dissipation of the bolus, to obtain a lower cardiac output that changes a frequency profile of the thermodilution curve. The processor 206 is configured to detect an increase in a mean signal from the ultrasonic transducer system 106 due to the cardiac output being lower with a same frequency of temperature increase of blood and the thermal dissipation of the bolus being below a baseline. Upon detecting an increase in the mean signal, the processor 206 is configured to shift the frequency of temperature increase and dissipation.

In accordance with an embodiment, the ultrasound transducer system 106 in configured to change the temperature upstream of the sensing modality using focused ultrasound technology, by means of creation of a 'bolus' with a different temperature.

For instance, HIFU creates a local high temperature in a tumorous tissue in order to create tissue damage. The increase of temperature is localized and dissipates rapidly and is close to ambient condition 8 mm away from the focal point. Taking into account that the size ranges of the right atrial long-axis and short-axis are 3.4-5.3 cm and 2.6-4.4 cm, respectively, the size of the aorta is 2.0 to 3.0 centimetres and the diameter of the superior vena cava in adults is 2.1 cm ± 0.7, the HIFU technology can increase the temperature in the bloodstream without heating up the surrounding tissue. Since the blood itself is mixing and flowing, the heat will dissipate and any long-term heating effect will be limited. The measurement of heat dissipation is done using ultrasound thermography.

In accordance with another embodiment, the cardiac output is measured by the thermal dissipation of the 'bolus' due to blood stream in the heart. By placing the HIFU focal point and the thermography measurement point closer to each other, the thermodilution profile amplitude is increased. In some instances, the thermodilution or the thermodissipation is measured, when combining the thermal increase and measurement into one location. This provides information of the flow rate of the various chambers of the heart.

In accordance with yet another embodiment, a cold saline bolus for thermodilution is used in combination with the ultrasound thermography described above.

In accordance with yet another embodiment, a thermodilution frequency is used to increase sensitivity. The thermodilution profile shows a sharp peak followed by a slow dissipation of the temperature due to the blood flow (given the CO). When given a 0°C cold saline bolus, the temperature difference between blood is large and a sharp profile may be seen. When using HIFU, a local area of the blood flow is heated up. However, the body is more sensitive to heat increase than a lower temperature. With the technology described in the present disclosure, the thermodilution profile is of a lower amplitude compared to currently known cold saline bolus methods.

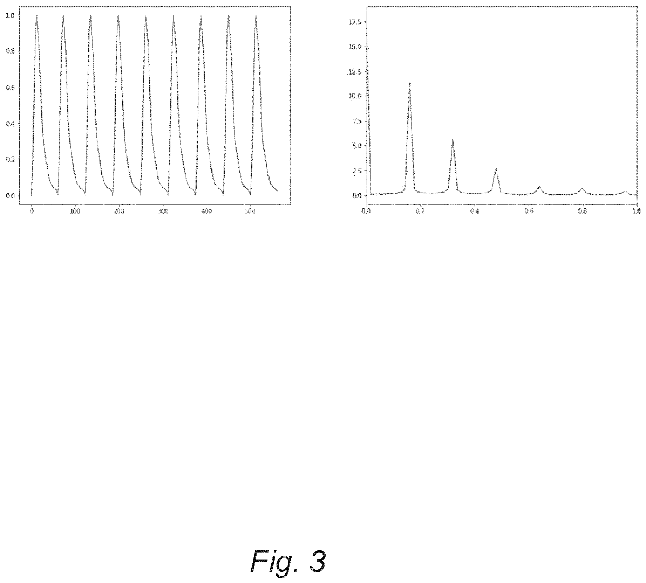

By using the thermodilution technique of the present disclosure, a higher measurement frequency may be achieved. By using the HIFU heating and ultrasound thermography in a repeated way, the resulting thermodilution curve will reflect a frequency profile, which can be analysed using FFT signal processing methods. For example, when the CO is high enough to create a flow to fully dissipate the temperature 'injection', a curve and FFT as illustrated in **FIG. 3** may be obtained.

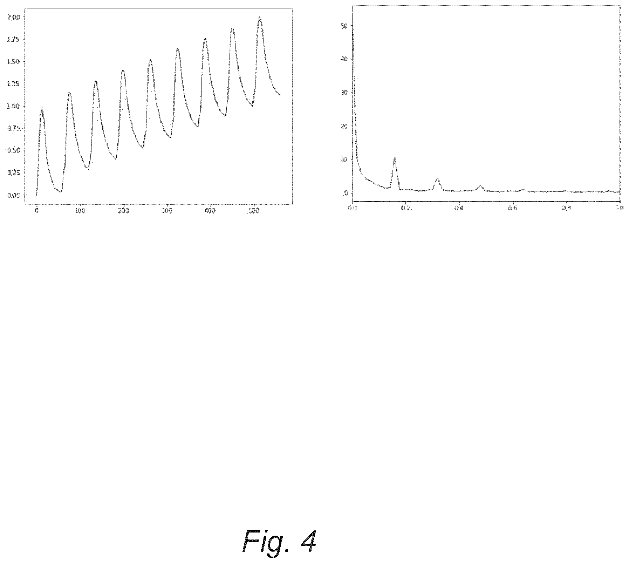

When the CO is lower with the same 'temperature injection' frequency, the thermal dissipation is not going to the baseline which results in a higher mean signal, which can be seen in the FFT plot at 0 Hz as illustrated in **FIG. 4****.** The processor 206 detects this increase and shifts the 'temperature injection' frequency to keep the system in equilibrium.

**FIG. 3** and **FIG. 4** illustrate thermodilution curves in accordance with various embodiments of the disclosure.

Referring to **FIG. 3****,** there is shown an illustration of a normal thermodilution curve (left) with the corresponding FFT (right) showing the frequency of the 'temperature injection' as the first peak (1 per minute, 0.01667 per second) and the mean signal, or the "bias" of the signal at 0 Hz.

Referring to **FIG. 4****,** there is shown an illustration of a low CO thermodilution curve (left) with the corresponding FFT (right) showing the frequency of the 'temperature injection' as the first peak (1 per minute, 0.01667 per second) and the mean signal, or the "bias" of the signal at 0 Hz. This bias is higher as depicted in FIG. 3 due to the incomplete thermal dissipation between the 'temperature injections'.

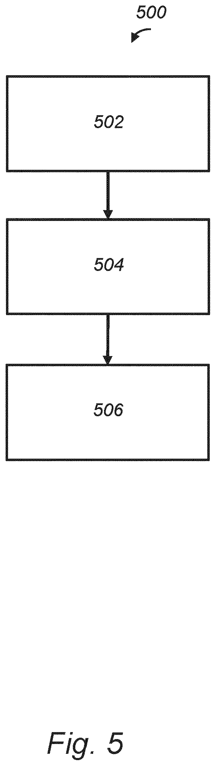

**FIG. 5** is a flowchart illustrating a method for performing non-invasive thermodilution in accordance with an exemplary embodiment of the disclosure. Referring to FIG. 5, there is shown a flowchart of a method 500 for performing non-invasive thermodilution using the apparatus 104 in accordance with an exemplary embodiment of the disclosure.

At **502,** locally increase the temperature of blood of a subject using high-intensity focused ultrasound (HIFU). The ultrasound transducer system 106 is configured to locally increase the temperature of the blood of the subject 102 using HIFU.

At **504,** measure a corresponding change in temperature of the blood downstream using ultrasonic thermometry. The ultrasound transducer system 106 is further configured to measure a corresponding change in temperature of the blood downstream using ultrasonic thermometry.

At **506,** create a thermodilution curve based on a measure of the change in temperature. The processing system 108 is configured to create the thermodilution curve 112 on the user interface 110, based on receiving signals pertaining to the measure of the change in temperature from the ultrasound transducer system 106.

The present disclosure may be realized in hardware, or a combination of hardware and software. The present disclosure may be realized in a centralized fashion, in at least one computer system, or in a distributed fashion, where different elements may be spread across several interconnected computer systems. A computer system or other apparatus/devices adapted to carry out the methods described herein may be suited. A combination of hardware and software may be a general-purpose computer system with a computer program that, when loaded and executed on the computer system, may control the computer system such that it carries out the methods described herein. The present disclosure may be realized in hardware that comprises a portion of an integrated circuit that also performs other functions. The present disclosure may also be realized as a firmware which form part of the media rendering device.

The present disclosure may also be embedded in a computer program product, which includes all the features that enable the implementation of the methods described herein, and which when loaded and/or executed on a computer system may be configured to carry out these methods. Computer program, in the present context, means any expression, in any language, code or notation, of a set of instructions intended to cause a system with information processing capability to perform a particular function either directly, or after either or both of the following: a) conversion to another language, code or notation; b) reproduction in a different material form.

While the present disclosure is described with reference to certain embodiments, it will be understood by those skilled in the art that various changes may be made, and equivalents may be substituted without departure from the scope of the present disclosure. In addition, many modifications may be made to adapt a particular situation or material to the teachings of the present disclosure without departure from its scope. Therefore, it is intended that the present disclosure is not limited to the particular embodiment disclosed, but that the present disclosure will include all embodiments that fall within the scope of the appended claims.

All definitions, as defined and used herein, should be understood to control over dictionary definitions, definitions in documents incorporated by reference, and/or ordinary meanings of the defined terms.

The subject matter described herein sometimes illustrates different components contained within, or connected with, different other components. It is to be understood that such depicted architectures are merely exemplary, and that in fact many other architectures can be implemented which achieve the same functionality. In a conceptual sense, any arrangement of components to achieve the same functionality is effectively "associated" such that the desired functionality is achieved. Hence, any two components herein combined to achieve a particular functionality can be seen as "associated with" each other such that the desired functionality is achieved, irrespective of architectures or intermedial components. The term "coupled" may be used herein to refer to any type of relationship, direct or indirect, between the components in question, and may apply to electrical, mechanical, fluid, optical, electromagnetic, electromechanical, or other connections. Likewise, any two components so associated can also be viewed as being "operably connected", or "operably coupled", to each other to achieve the desired functionality, and any two components capable of being so associated can also be viewed as being "operably couplable", to each other to achieve the desired functionality. Specific examples of operably couplable include but are not limited to physically mateable and/or physically interacting components.

In the claims, as well as in the specification above, the terms "first", "second", etc. may be used herein only to facilitate discussion, and carry no particular temporal or chronological significance unless otherwise indicated.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

The indefinite articles "a" and "an," as used herein in the specification and in the claims, unless clearly indicated to the contrary, should be understood to mean "at least one."

As used herein, the term "or" or "and/or" is inclusive and not exclusive, unless expressly indicated otherwise or indicated otherwise by context. Therefore, herein, "A or B" means "A, B, or both," unless expressly indicated otherwise or indicated otherwise by context. Moreover, "and" is both joint and several, unless expressly indicated otherwise or indicated otherwise by context. Therefore, herein, "A and B" means "A and B, jointly or severally," unless expressly indicated otherwise or indicated otherwise by context.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

As used in this application and in the claims, a list of items joined by the term "one or more of" may mean any combination of the listed terms. For example, the phrases "one or more of A, B or C" may mean A; B; C; A and B; A and C; B and C; or A, B and C.

As is described above in greater detail, one or more processor, other unit, the like, and/or combinations thereof may fulfill the functions of several items recited in the claims.

As is described above in greater detail, a computer program may be stored/distributed on a suitable medium, such as an optical storage medium or a solid-state medium supplied together with or as part of other hardware, but may also be distributed in other forms, such as via the Internet or other wired or wireless telecommunication systems.

It should also be understood that, unless clearly indicated to the contrary, in any methods discussed herein that include more than one step or act, the order of the steps or acts of the method is not necessarily limited to the order in which the steps or acts of the method are recited. Further, such methods may include additional or alternative steps or acts. As used in the claims, the mere fact that certain measures are recited in mutually different dependent claims does not indicate that a combination of these measured cannot be used to advantage.

Those skilled in the art will appreciate from the foregoing description that the broad techniques of the embodiments of the present disclosure can be implemented in a variety of forms. Therefore, while the embodiments of this disclosure have been described in connection with particular examples thereof, the true scope of the embodiments of the disclosure should not be so limited since other modifications will become apparent to the skilled practitioner upon a study of the drawings, specification, and following claims.

## Claims

1. An apparatus for performing non-invasive thermodilution, comprising:
an ultrasonic transducer system comprising one or more ultrasonic transducers or transducer arrays, the ultrasonic transducer system configured to:
locally increase the temperature of blood of a subject using high-intensity focused ultrasound (HIFU); and
measure a corresponding change in temperature of the blood downstream using ultrasonic thermometry; and
a processor communicatively coupled to the ultrasonic transducer system, wherein the processor is configured to create a thermodilution curve based on receiving signals pertaining to the measure of the change in temperature from the ultrasound transducer system.

2. The apparatus of claim 1, wherein the ultrasonic transducer system is configured to administer a bolus into bloodstream of the subject to locally increase the temperature of the blood using HIFU.

3. The apparatus of claim 2, wherein the bolus is a cold saline solution.

4. The apparatus of claim 2, wherein a temperature of the bolus is different from a temperature of the bloodstream of the subject.

5. The apparatus of claim 1, wherein the ultrasonic transducer system measures a blood flow rate of the subject.

6. The apparatus of claim 1, wherein the processor is configured to derive a cardiac output of the subject from the thermodilution curve based on a thermal dissipation of the bolus due to bloodstream in the heart of the subject.

7. The apparatus of claim 1, wherein the processor is configured to analyze the thermodilution curve using a frequency profile based on signal processing.

8. The apparatus of claim 7, wherein the processor is configured to analyze the thermodilution curve using one of a Fast Fourier Transform (FFT) signal processing and an impulse response function.

9. The apparatus of claim 1, wherein a thermodilution profile amplitude of the thermodilution curve is increased by placing an HIFU focal point and a thermometry measurement point closer to each other.

10. The apparatus of claim 2, wherein a frequency of temperature increase and dissipation is chosen such that the temperature of the blood is increased directly after complete thermal dissipation of the bolus, to obtain a lower cardiac output that changes a frequency profile of the thermodilution curve.

11. The apparatus of claim 10, wherein the processor is configured to detect an increase in a mean signal from the ultrasonic transducer system due to the cardiac output being lower with a same frequency of temperature increase of blood and the thermal dissipation of the bolus being below a baseline, and wherein upon detecting an increase in the mean signal, the processor is configured to shift the frequency of temperature increase and dissipation.

12. A method for performing non-invasive thermodilution, comprising:
locally increasing the temperature of blood of a subject using high-intensity focused ultrasound (HIFU);
measuring a corresponding change in temperature of the blood downstream using ultrasonic thermometry; and
creating a thermodilution curve based on a measure of the change in temperature.

13. The method of claim 12, wherein locally increasing the temperature of the blood using HIFU comprises administering a bolus into the bloodstream of the subject.

14. The method of claim 13, comprising choosing a frequency of temperature increase and dissipation such that the temperature of the blood is increased directly after complete thermal dissipation of the bolus, to obtain a lower cardiac output that changes a frequency profile of the thermodilution curve.

15. The method of claim 14, comprising detecting an increase in a mean signal due to the cardiac output being lower with a same frequency of temperature increase of blood and the thermal dissipation of the bolus being below a baseline, and wherein upon detecting an increase in the mean signal, shifting the frequency of temperature increase and dissipation.
